# EUROPEAN PATENT SPECIFICATION

(11) **EP 0 518 071 B1**
(45) Date of publication and mention of the grant of the patent: **26.10.1994**
(21) Application number: 92108118.8
(22) Date of filing: 14.05.1992
(51) Int. Cl.: A61B 17/16, A61B 17/58

(54) **Device for making holes for the implantation of interlocking nails**
Vorrichtung zum Bohren von Löchern zur Implantation von Verriegelungsnägeln für Marknägel
Dispositif à faire des trous pour l'implantation des vis de verrouillage des clous intramedullaires

(30) Priority: 13.06.1991 DE 9107298 U
(43) Date of publication of application: 16.12.1992
(73) Proprietor: Howmedica GmbH, D-24232 Schönkirchen (DE)
(72) Inventor: Ritter, Prof. Dr., W-6500 Mainz (DE)
(74) Representative: Patentanwälte Hauck, Graalfs, Wehnert, Döring, Siemons

(56) References cited:
- EP-A- 0 167 719
- EP-A- 0 201 737
- EP-A- 0 281 763
- EP-A- 0 358 579
- US-A- 4 722 336
- US-A- 4 848 327

## Description

The invention refers to a device for making holes for the implantation of interlocking nails.

Interlocking nails are used for bone fractures and have transverse bores for a fixation within the bone by bone screws to be secured against relative axial and rotational movement. The interlocking nails are proximally inserted and the distal portion thereof normally has two transverse bores so that aligned bores or holes must be made in the corticalis. The detection of the position of the transverse bores within the bone is relatively difficult and requires additional means. For this purpose, target devices are used in combination with an X-ray source and an X-ray image converter.

Manually operable unsupported target devices are known as well as target devices attached to a relatively stationary target system. The attachment of the target device immediately to an image converter enables an accurate setting of holes. A displacement of the leg of a patient or of the surgical table causes a misalignment.

The German Gebrauchsmuster 84 17 428 corresponding to the preamble of claim 1 discloses to combine a target device with a power-driven drilling tool in that a radiation transparent chuck is used. The drill bit is projected as a spot if it is extended parallel to the radiation direction.

The EP-A-0 201 737 discloses a target device including a drill sleeve connected to a handle and sighting means connected to the sleeve, with the position of the sighting means between a radiation source and a radiation receiver made visible through an image converter. Sighting means separate from the drilling sleeve have the advantage that a control and a correction of the target device can be carried out during the drilling process. However, it is disadvantageous that a target element must be attached to the drilling sleeve prior to the detection of the drilling axis by means of the sighting means. A target element may consist of a radiation transparent pin having a radiation-impervious spot at the tip. By means of the sighting means and the target pin, the drill sleeve can be accurately positioned against the bone. After a repeated alignment with the sighting means the setting of the hole is carried out by guiding the drill bit in the drilling sleeve.

The German Gebrauchsmuster 87 03 438 discloses an auxiliary instrument for the setting of holes wherein a guiding member is attached to a handle and is made of a material transparent for X-rays. The guiding member includes a guiding bore for the slidable guidance of a rod-shaped punching tool. The guiding member includes two axially spaced annular members encircling the guiding bore which appear in an overlapped formationon the screen if the X-rays are aligned with the guiding bore.

The described manually operable devices require a permanent correction of the position of the drilling sleeve during the drilling process in order to secure that the first bore in the corticalis as well as the second one have the accurate position. This correction is made by the X-ray means.

It is an object of the invention to provide a device for setting holes for the implantation of interlocking nails which can be easily handled, allows an exact setting of the holes and reduces the exposure rate of the surgeon.

The solution to the problem posed is given by the characterising features of claim 1 whereby the device according to the invention includes a corner drill having a coupling fitting opposite to the chuck for the accommodation of a telescopic guide member. The free end of the guide member is adapted to engage the window of the image converter or the front portion of the housing of the X-ray source such that the axis of the guide member is coaxial with the beam axis.

The device according to the invention makes use of the fact that it is relatively simple to detect the point on the corticalis through which the axis of both coaxial locking bores extends. This point on the bone can be marked in a suitable manner, e.g. by means of a bone pin or a drill bit. For example, the corticalis can be spot drilled at this point to secure that the drill bit does not slide away when the first and the second corticalis are drilled. It is decisive that the drill bit is guided along the axis through the locking bores in that the drilling tool with the drill bit is aligned accordingly, with the drill bit engaging the mentioned spot. This is achieved with the aid of the telescopic guiding member which can be relatively rigidly connected to the corner drill while its other end engages the window of the image converter or the front housing portion of the X-ray source. For the sake of explanation, it is to be noted that the X-ray source may be directly radiate on the operation area or from the opposite side in dependence of the method used. The image converter is placed accordingly. If the X-ray source is located above the operation area, the guide member is brought into engagement with the housing of the X-ray source. Conventional X-ray sources have a conical front portion. Accordingly, the guide member has an inner cone to be plugged on the outer cone of the housing. Since the axis interconnecting the X-ray source and the image converter is aligned with the axis of the coaxial locking bores (this is mandatory for the precise setting of the holes for the implantation of interlocking nails), the drilling tool is aligned correspondingly and is permanently guided during the drilling process since the telescopic guide member is extended longitudinally during the drilling process and maintains its guiding properties.

If, however, the image converter faces the operation area, an engagement surface at the free end of the guide member engages the window of the image converter. To this purpose, the telescopic guide member is extended up to the window of the image converter, and the assistant is looking for the desired orientation of the guide member wherein the engagement surface engages the window flush. In this case, the drilling tool is precisely aligned and the drilling process can be started. Appropriately, the guide member is continuously held into engagement with the window of the image converter during the drilling process.

From the above explanation of the function of the device according to the invention, it can be seen that the X-ray source must not be switched on during the alignment process of the guide member and the drilling process. Thus, the surgeon is not subject to any X-ray beams during these process steps.

From the above-described target device it is known to provide a sleeve-like target member which also serves for the guidance of the drill bit. However, in order to find out the engagement point on the first corticalis for the drill bit the target sleeve must be exactly aligned with the X-ray beam with respect to all planes. In case the sleeve is not accurately aligned, it is impossible to align the bore of the sleeve with respect to the holes in the interlocking nail accurately. In case this engagement point has been found, the next problem is to not displace the sleeve during the drilling process and to maintain its position during the insertion of the drill bit and during the drilling process because no control can be carried out during the drilling process. Therefore, an embodiment of the invention provides a sleeve-like target member having an upper portion which tapers conically upwardly while the lower opening corresponds to the outer diameter of the drill bit.

Therefore, the invention does not use a cylindrical drilling sleeve, rather, a kind of funnel. With such a conical drilling sleeve only the exit of the funnel has to be aligned with the bores of the nail on the monitor of the X-ray image converter while the other position or attitude of the funnel can be disregarded.

The surgical operation is relatively simple. After the soft tissue incision has been made, the funnel is first brought into engagement with the bone. Thereafter, the image converter is switched on. The exit opening of the funnel can be simply aligned with the locking bores. In case the opening of the funnel on the monitor is circular, the lower opening of the funnel is exactly aligned with the locking bores of the nail. Thus, the engagement point for the drill bit has been detected. This targeting process requires only a few seconds and is the single phase wherein an X-ray illumination is necessary for the setting of the holes. The further steps do not require any X-rays. The surgeons are not subject to an X-ray load. After the positioning of the funnel, the drill bit is introduced by means of the corner drill. The exact angular position of the corner drill can be disregarded. The bone is merely spot-drilled in order to achieve an engagement point for the major drilling process. This short spot-drilling can be made during a period of 2 to 3 seconds.

According to an embodiment of the invention, the target member is cylindrical above the conical portion, and an accommodation sleeve for a guiding sleeve is located between the handle and the target member. The axis of the accommodation sleeve has a distance from the axis of the target member which corresponds to the space between the pairs of locking bores. With the aid of a guiding bushing introduced into the accommodation sleeve, the first corticalis can be spot-drilled with respect to the second pair of locking bores.

Embodiments of the invention are subsequently explained along accompanied drawings.
- Fig. 1: is a diagrammatic perspective view of the target device according to the invention.
- Fig. 2: is a side view of a device of the invention during a first operational phase.
- Fig. 3: is a similar view as Fig. 2 in a successive operational phase.
- Fig. 4: is a view of the device of Fig. 2 during the drilling phase.
- Fig. 5: is a view similar to Fig. 2 with a slight modification.
- Fig. 6: is a diagrammatic view of the monitor showing the alignment of the target member with respect to an interlocking nail.
- Fig. 7: is a side view of a modified target device.

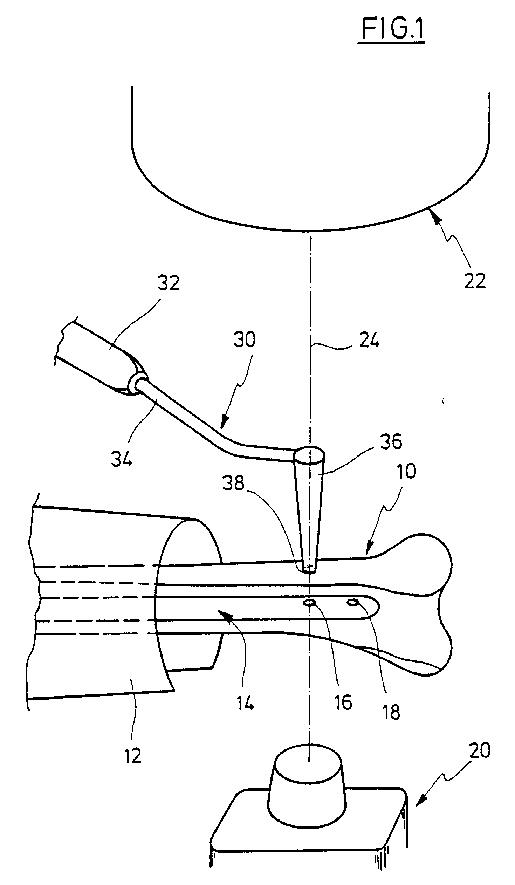

In Fig. 1, a distal femur portion 10 of a patient on an operation table (not shown) can be seen. The proximal area of the leg is covered by a sheet 12 of lead. An interlocking nail 14 is introduced into the femur 10 proximally, the nail 14 including two pairs of distal locking bores 16, 18. Such an interlocking nail is conventional.

An X-ray device includes an X-ray source 20 below the femur 10 and an image converter 22 above the femur. The axes of the image converter 22 and of the X-ray source 20 are aligned as indicated by the dashed line on a beam axis 24. X-ray source 20 and image converter 22 are rigidly interconnected. For the setting of holes in the corticalis of the femur 10 they are aligned such that the beam axis 24 extends through both bores of a pair of bores 16, 18, respectively (this is the case when both locking bores of a pair overlap each other and appear on the window of the image converter 22 or the respective monitor, respectively, as a circle).

A target device 30 has a handle 32, a shank 34 and a conical target sleeve 36 at the end of the shank. The edge of the lower opening of sleeve 36 is toothed as indicated at 38. The diameter of the lower opening of the sleeve 36 is slightly larger than the outer diameter of the drill bit by which the corticalis is to be bored.

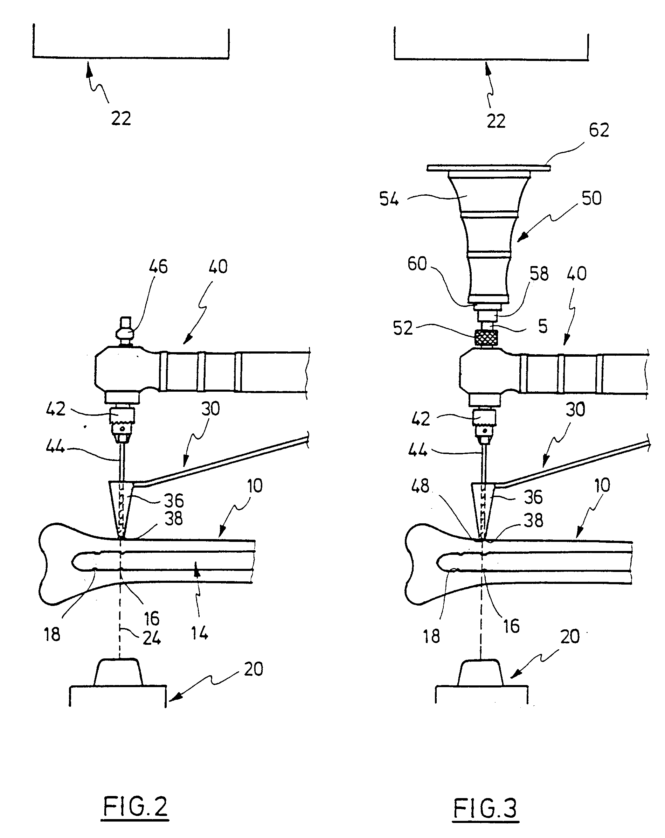

After the incision of the soft tissue is made, the target sleeve 36 is brought into engagement with the bone. Thereafter, the X-ray source 20 and the image converter 22 are switched on. By observation of the monitor (not shown), the lower edge 38 of sleeve 36 can be brought into alignment with the locking bores. If the opening of sleeve 36 is circular and open, it is exactly aligned with a respective pair of bores 16 or 18 of nail 14. For this targeting which needs 3 to 10 seconds at the maximum an X-ray illumination is necessary. Thereafter, the corticalis is spot-drilled. This can be seen in the Figures 2 and 3. A corner drill 40 is located below the image converter 22. A drill bit 44 is clamped in a chuck 42. On the end opposite to the chuck 42 a quick-coupling fitting 46 is located. The drill bit 44 is inserted into the target sleeve 36 in order to spot-drill the femur 10 as indicated in Fig. 3 at 48. During this process, the exact angular position of the corner drill can be disregarded.

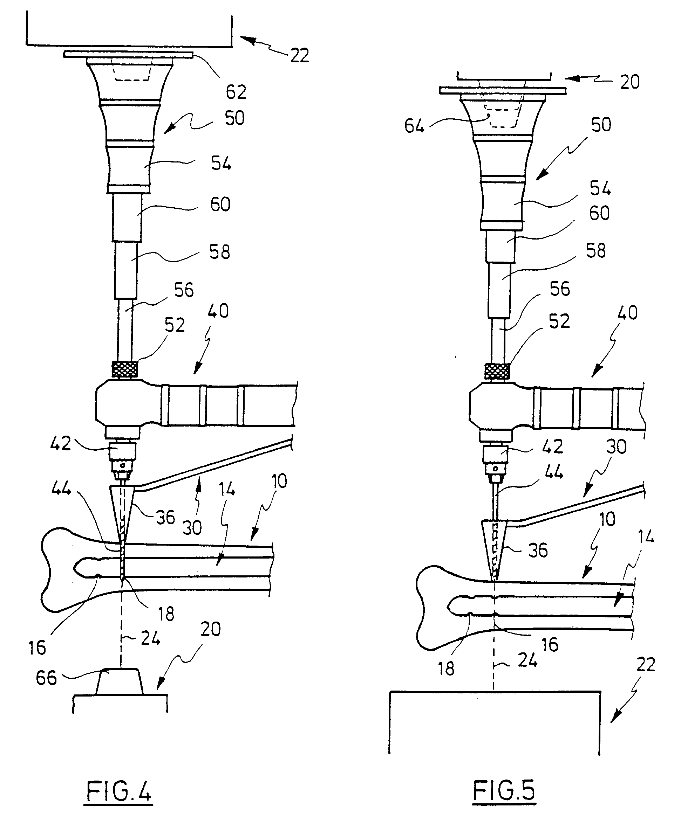

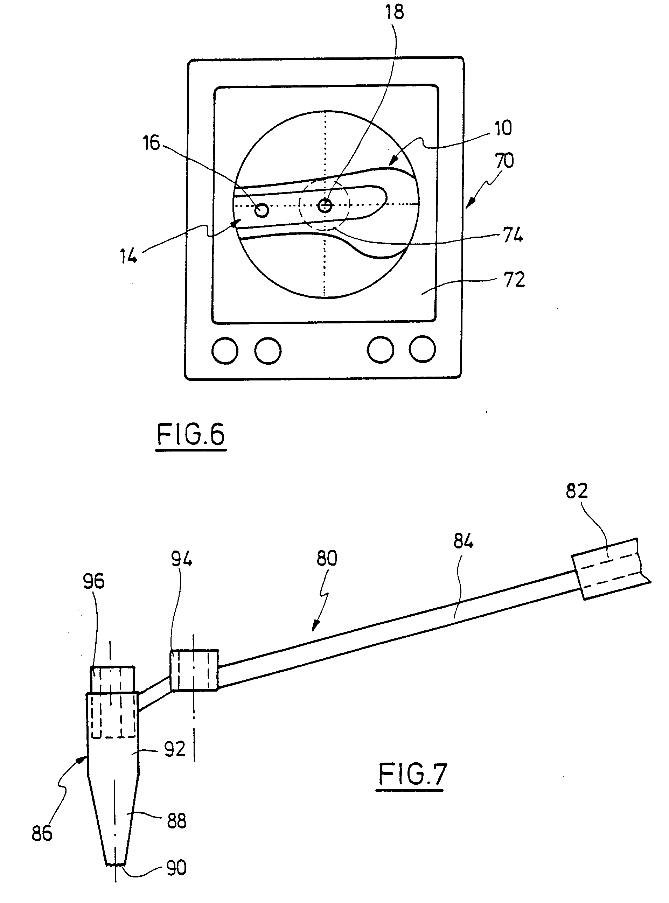

A telescopic guide member 50 is coupled with the corner drill 40. This takes place by means of a coupling portion 52 coacting with the coupling fitting 46. As can be seen in Figs. 4 and 5, the guide member 50 includes a receiving or retaining conical portion 54 and three cylindrical tubular portions 56, 58 and 60 as well which can be telescopically pushed together and accommodated by the conical portion 54 as can be seen in Fig. 3. At the free end of the conical portion 54, an annular engagement disc 62 is provided defining a flat engagement surface. In the area of the engagement disc 62, an inner cone 64 is provided in the conical portion 54 as can be seen in Fig. 5.

When the guide member 50 is attached to the corner drill 40 as shown in Fig. 3, the guide member 50 is telescopically extended as shown in Fig. 4. The engagement disc 62 is brought into engagement with the entrance window of the image converter 22 such that it lies completely against the window over its circumference.

Thereby, the corner drill 40 has a position wherein the axis of the drill bit 44 is coaxial with the beam axis 24. When the drill bit 44 is pierced through the first corticalis as shown in Fig. 4, it can be extended through both locking bores 18 and can drill the second corticalis subsequently. During this process, the guide member 50 is extended without losing its guiding function. For this, it is mandatory that the engagement disc 62 is completely held against the window of image converter 22.

In some systems, the location of the X-ray source and the image converter 22 is inversed. This is shown in Fig. 5. It can be seen that the housing of the X-ray source has a conical portion 66 at the front end. The inner cone 64 of conical portion 54 of guide member 50 fits to the conical housing portion 66 so that an alignment of the guide member 50 with respect to the axis 24 of the X-ray device can be also achieved.

In Fig. 6 an image of the femur and of the interlocking nail on a window 72 of a monitor 70 is shown. A sheet 74 bearing a circle having for example a diameter of 50 mm is sticked to the window in the center thereof. If it is taken care that both pairs of bores 16, 18 of nail 14 are lying on a diameter within the circle, the image converter device is sufficiently aligned with respect to the interlocking nail 14. Then, the process described above can be carried out for both pairs of locking bores 16, 18.

In order to carry out a spot drill of the femur at two spaced locations, a manually operable target device 80 of Fig. 7 is provided having a handle 82, a shank 84 and a target sleeve 86. The target sleeve has a conical portion 88, with a lower opening having a toothed edge 90. A cylindrical portion 92 is located above the conical portion 88.

An accommodation sleeve 94 spaced from the target sleeve 92 is located at shank 84. The axis of the target sleeve 86 and of the accommodation sleeve 94 are spaced from each other, with the space corresponding to the distance between the locking bores 16, 18. The first spot drill can be made with the target device 80 of Fig. 7 in the same manner as described in connection with Figs. 1 to 5. The drilling through the corticalis by means of the target sleeve takes place in the manner explained above. The drill bit extending through the corticalis and the bores in the interlocking nail can be removed from the corner drill and still extend through the target sleeve 86. An auxiliary target sleeve 96 is slided over the drill bit (not shown), the outer diameter of the auxiliary sleeve 96 corresponding to the inner diameter of the cylindrical portion 92 while the inner diameter corresponds to the outer diameter of the drill bit. In case, the auxiliary sleeve 96 is inserted into the target sleeve 86, the target sleeve 86 is coaxial to the axis of the locking bores. Then, the accommodation sleeve 94 has a distance from the common axis which corresponds to the distance between the pairs of locking bores. An alignment of the accommodation sleeve in a plane perpendicular to this axis takes place by the X-ray device. To this purpose, the X-ray source is switched on again so that an aligning process can take place within a short time in order to drill the corticalis in alignment with the second pair of locking bores.

A pin can be used instead of a drill bit which is inserted through the holes in the corticalis and the first pair of locking bores in order to achieve an axial alignment of the target sleeve 96 by means of the auxiliary target sleeve 96.

## Claims

1. A device for making holes for the implantation of interlocking nails (14) having distal locking bores (16, 18), comprising an X-ray source (20) having a housing and a beam axis (24) adapted to be aligned with said locking bores and an associated image converter having an entrance window, said device comprising further a drilling tool for drilling the corticalis coaxially with said beam axis (24) and guide means for guiding said drilling tool, characterized in that a corner drill (40) is provided having a chuck (42) and a coupling fitting (46) opposite to said chuck and that said guide means comprises a telescopic guide member (50) which provides axial guiding of the drilling tool during extension, and is adapted to be coupled to said fitting (46) with one end and to engage said window of said image converter (22) by its opposite end or a front portion of said housing of said X-ray source, respectively, such that the axis of said guide member (50) is coaxial with said beam axis (24).

2. The device of claim 1, wherein an engagement disc (62) is located at the free end of said guide member (50).

3. The device of claim 1 or 2, wherein an inner cone (64) is provided at the free end of said guide member (50) for the receipt of a conical portion (66) of said housing of said X-ray source (20).

4. The device of claim 1, wherein said coupling fitting (46) of said corner drill (40) and said end of said guide member (50) to be coupled with said coupling fitting (46) define a quick connection coupling.

5. The device of claim 1 including a handle with a sleeve-like target member wherein said sleeve-like target member (36, 86) has a lower opening (38) with a diameter corresponding to the outer diameter of said drill bit (44), said target member (36, 86) tapering conically upwardly.

6. The device of claim 5, wherein said target member (86) has a cylindrical portion (92) above said conical enlargement (88) and an accommodation sleeve (94) for a guide sleeve is located between said handle (82) and said target member (86)

7. The device of claim 6, wherein an auxiliary target sleeve (96) is provided, with the outer diameter thereof corresponding to the inner diameter of said cylindrical portion (92) and the inner diameter thereof corresponding to the outer diameter of said drill bit or a pin, respectively.

## Patentansprüche

1. Vorrichtung zum Setzen von Löchern bei der Implantation von Verriegelungsnägeln (14) mit distalen Verriegelungslöchern (16, 18), wobei die Vorrichtung einen Röntgenstrahler (20) mit einem Gehäuse und einer zu den Verriegelungslöchern ausrichtbaren Strahlachse (24) und einen zugehörigen Bildwandler mit einem Eintrittsfenster aufweist, die Vorrichtung weiterhin ein Bohrwerkzeug zum Bohren der Corticalis koaxial zur Strahlachse (24) und ein Führungsorgan zum Führen des Bohrwerkzeugs aufweist, dadurch gekennzeichnet, daß ein Winkelbohrer (40) mit einem Bohrfutter (42) und einem Kupplungsabschnitt (46) auf der dem Bohrfutter entgegengesetzten Seite vorgesehen ist und daß ein teleskopartiges Führungsorgan (50) das Bohrwerkzeug während des Auszugs axial führt und mit einem Ende an den Kupplungsabschnitt (46) kuppelt und mit seinem entgegengesetzten Ende auf der Fensterebene des Bildwandlers (22) oder auf dem vorderen Gehäuseabschnitt des Röntgenstrahlers so aufsetzbar ist, daß die Achse des Führungsorgans (50) koaxial mit der Strahlachse (24) verläuft.

2. Vorrichtung nach Anspruch 1, dadurch gekennzeichnet, daß am freien Ende des Führungsorgans (50) ein Aufsetzteller (62) angeordnet ist.

3. Vorrichtung nach Anspruch 1 oder 2, dadurch gekennzeichnet, daß am freien Ende des Führungsorgans (50) ein Innenkonus (64) angeordnet ist für die Aufnahme eines konischen Abschnitts (66) des Gehäuses des Röntgenstrahlers (20).

4. Vorrichtung nach Anspruch 1, dadurch gekennzeichnet, daß der Kupplungsabschnitt (46) an der Winkelbohrmaschine (40) und das aufsteckbare Ende des Führungsorgans (50) eine Schnellkupplung bilden.

5. Vorrichtung mit einem an einer Handhabe angebrachten hülsenförmigen Zielorgan nach Anspruch 1, dadurch gekennzeichnet, daß das hülsenförmige Zielorgan (36, 86) oberhalb der unteren Öffnung (38), deren Durchmesser dem Außendurchmesser des Bohrers (44) entspricht, konisch erweitert ist.

6. Vorrichtung nach Anspruch 5, dadurch gekennzeichnet, daß das Zielorgan (86) oberhalb der konischen Erweiterung (88) zylinderförmig (92) ist und zwischen dem Handgriff (82) der Handhabe und dem Zielorgan (86) eine Aufnahmehülse (94) für eine Führungshülse angeordnet ist.

7. Vorrichtung nach Anspruch 6, dadurch gekennzeichnet, daß eine Hilfszielhülse (96) vorgesehen ist, deren Außendurchmesser dem Innendurchmesser des zylindrischen Abschnitts (92) und deren Innendurchmesser dem Außendurchmesser eines Bohrers oder eines Stifts entspricht.

## Revendications

1. Dispositif pour percer des trous pour l'implantation de clous de verrouillage réciproque (14) comportant des trous de blocage distaux (16, 18), ledit dispositif comprenant une source de rayons X (20), qui comporte un boitier et un axe de faisceau (24) conçu pour être aligné avec lesdits trous de blocage, et un convertisseur d'image associé possédant une fenêtre d'entrée, ledit dispositif comprenant également un outil de perçage pour percer la corticale coaxialement audit axe de faisceau (24) et un moyen de guidage pour guider ledit outil de perçage, caractérisé en ce qu'il est prévu une perceuse à angle (40) possédant un mandrin (42) et un raccord d'accouplement (46) situé à l'opposé dudit mandrin, et en ce que ledit moyen de guidage comprend un élément de guidage télescopique (50) qui assure le guidage axial de l'outil de perçage lors de l'extension et qui est conçu pour être relié audit raccord (46) par l'une de ses extrémités et pour venir au contact de ladite fenêtre dudit convertisseur d'image (22) par son extrémité opposée ou, respectivement, contre une partie avant dudit boîtier de ladite source de rayons X, de façon que l'axe dudit élément de guidage (50) soit coaxial audit axe de faisceau (24).

2. Dispositif selon la revendication 1, dans lequel un disque de contact (62) est situé à l'extrémité libre dudit élément de guidage (50).

3. Dispositif selon la revendication 1 ou 2, dans lequel un cône intérieur (64) est prévu à l'extrémité libre dudit élément de guidage (50) pour recevoir une partie conique (66) dudit boitier de ladite source de rayons X (20).

4. Dispositif selon la revendication 1, dans lequel ledit raccord d'accouplement (46) de ladite perceuse à angle (40) et ladite extrémité dudit élément de guidage (50) destinée à être reliée audit raccord d'accouplement (46) forment un accouplement à liaison rapide.

5. Dispositif selon la revendication 1, comprenant une poignée munie d'un élément formant cible en forme de manchon, dans lequel ledit élément formant cible en forme de manchon (36, 86) comporte une ouverture inférieure (38) possédant un diamètre correspondant au diamètre extérieur dudit foret (44), ledit élément formant cible (36, 86) étant évasé vers le haut en forme de cône.

6. Dispositif selon la revendication 5, dans lequel ledit élément formant cible (86) comporte une partie cylindrique (92) située au-dessus de l'élargissement conique (88), et un manchon de logement (94) destiné à un manchon de guidage est disposé entre ladite poignée (82) et ledit élément formant cible (86).

7. Dispositif selon la revendication 6, dans lequel il est prévu un manchon auxiliaire formant cible (96), dont le diamètre extérieur correspond au diamètre intérieur de ladite partie cylindrique (92) et dont le diamètre intérieur correspond au diamètre extérieur dudit foret ou, respectivement, d'une broche.
